# EUROPEAN PATENT SPECIFICATION

(11) **EP 3 266 384 B1**
(45) Date of publication and mention of the grant of the patent: **01.04.2020**
(21) Application number: 17185817.8
(22) Date of filing: 06.08.2013
(51) Int. Cl.: A61B 17/00, A61B 17/064, A61B 17/11, A61B 17/12

(54) **DEVICES FOR ENGAGING TISSUE**
VORRICHTUNGEN ZUM EINGRIFF IN EIN GEWEBE
DISPOSITIFS DE FIXATION À UN TISSU

(30) Priority: 10.08.2012 US 201261682141 P; 06.03.2013 US 201361773442 P; 05.08.2013 US 201313958665
(43) Date of publication of application: 10.01.2018
(62) Divisional of application: 13759901.5
(73) Proprietor: W.L. Gore & Associates, Inc., Newark DE 19711 (US)
(72) Inventor: CULLY, Edward, H., Newark, Delaware 19711 (US); DUNCAN, Jeffrey, B, Newark, Delaware 19711 (US); SMITH, Benjamin, A, Newark, Delaware 19711 (US)
(74) Representative: HGF Limited

(56) References cited:
- US-A1- 2003 212 418
- US-A1- 2004 078 053
- US-A1- 2007 073 337
- US-A1- 2010 160 847

## Description

### FIELD

The present disclosure relates generally to medical devices and, more specifically, to anchors to secure medical devices to tissue in a patient.

### BACKGROUND

Medical devices are frequently used to treat the anatomy of patients. Such devices may be temporarily, semi-permanently, or permanently implanted in the anatomy to provide treatment to the patient. It is important that such devices maintain proper position within the anatomy. Therefore, it is desirable to provide devices, systems and methods for implanting and maintaining position of medical devices within the anatomy of a patient.

US 2003/212418 discloses a one piece anastomosis device which is formed of a superelastic or pseudoelastic material which self-deforms or self-deploys from an insertion configuration to a tissue holding configuration. The device in a deployed state preferably includes an inner tissue penetrating flange which penetrate and retains an everted graft vessel and an outer flange. The self deploying anastomosis device does not rely on a temperature transformation to achieve deployment.

US 2010/160847 discloses a cardiovascular conduit system may comprise a connector. The connector may comprise a proximal end adapted to attach to a cardiovascular organ. The proximal end may comprise a first plurality of expandable members, and each member in the first plurality of expandable members may be deployable from a delivery position to a deployed position. The first plurality of expandable members may be dimensioned to deploy inside the cardiovascular organ to secure the connector to the cardiovascular organ. The connector may comprise a distal end adapted to attach to a conduit and an opening extending through the connector. Connectors for cardiovascular conduit systems may also include expandable stents. Connectors may be rotateably secured to a conduit, and the conduit may be reinforced.

US 2004/ 078053 discloses a medical graft connector or plug which is made, for example, by cutting end portions of a tube of highly elastic material axially at numerous locations spaced circumferentially around the tube to produce a plurality of fingers which extend axially from each end of an uncut medial portion of the tube. The fingers are deflected radially outwardly from the medial portion and set in that condition. For a graft connector, the medial portion is coaxially connected to an end portion of a tubular graft. The connector is then installed through an aperture in the side wall of a patient's tubular body conduit, for example, by using a delivery tube in which the fingers are elastically deflected back to approximately their initial positions. When the delivery conduit is withdrawn from the connector, the fingers spring out to engage the inner and outer surfaces of the body conduit wall. For a plug, the medial portion is occluded and then the structure is installed through the aperture to be plugged in a manner similar to installation of the connector.

### BRIEF DESCRIPTION OF THE DRAWINGS

The accompanying drawings are included to provide a further understanding of the disclosure and constitute a part of this specification, illustrate embodiments and examples of the disclosure, and together with the description, serve to explain the principles of the disclosure, wherein;
Figures 1A and 1B illustrate cross sections of delivery systems in accordance with the present disclosure;
Figures 2A-2E illustrate perspective views of anchors in accordance with the present disclosure but not forming part of the present invention;
Figures 3A and 3B illustrate side views of tissue-penetrating points in accordance with the present disclosure;
Figures 4A and 4B illustrate side views of an anchor in accordance with the present disclosure;
Figures 5A and 5B illustrate a side and perspective view, respectively, of an anchor formation system in accordance with the present disclosure;
Figures 6A-6F illustrate medical devices comprising anchors not forming part of the present invention;
Figures 7A and 7B illustrate different stages of deployment of an anchor device not forming part of the present invention;
Figure 8 illustrates a flow chart of an anchor deployment method in accordance with the present disclosure;
Figures 9A-9C illustrate various stages of deployment of an anchor not forming part of the present invention;
Figures 10A and 10B different various stages of deployment of a plurality of anchors in accordance with the present disclosure;
Figures 11A-11C illustrate various stages of deployment of a medical device comprising an anchor not forming part of the present invention;
Figures 12A-12C illustrate various stages of deployment of a device comprising a plurality of anchors in accordance with the present disclosure; and
Figures 13A-13C illustrate a top view and two side views, respectively, of devices comprising a plurality of anchors in accordance with the present disclosure.
Figure 14 illustrates a perspective view of an embodiment of the present invention comprising a flange element having a cap of biomaterial.
Figures 15A and 15B illustrate a side view and a top view of an example comprising a flange element,

### DETAILED DESCRIPTION OF THE ILLUSTRATED EMBODIMENTS

Persons skilled in the art will readily appreciate that various aspects of the present disclosure can be realized by any number of methods and systems configured to perform the intended functions. Stated differently, other methods and systems can be incorporated herein to perform the intended functions. It should also be noted that the accompanying drawing figures referred to herein are not all drawn to scale, but can be exaggerated to illustrate various aspects of the present disclosure, and in that regard, the drawing figures should not be construed as limiting.

As used herein, the term "elongate element" is generally any element configured for relative axial movement with an endoluminal device delivery element (e.g., a catheter-based endoluminal device delivery element such as a balloon catheter) and includes any longitudinally extending structure with or without a lumen therethrough. Thus, elongate elements include but are not limited to tubes with lumens (e.g., catheters), solid rods, hollow or solid wires (e.g., guidewires), hollow or solid stylets, metal tubes (e.g., hypotubes), polymer tubes, pull cords or tethers, fibers, filaments, electrical conductors, radiopaque elements, radioactive elements and radiographic elements. Elongate elements can be any material and can have any cross-sectional shape including, but not limited to, profiles that are ellipitcal, non-ellipitcal, or random. Typical materials used to construct elongate element, such as catheters, can comprise commonly known materials such as Amorphous Commodity Thermoplastics that include Polymethyl Methacrylate (PMMA or Acrylic), Polystyrene (PS), Acrylonitrile Butadiene Styrene (ABS), Polyvinyl Chloride (PVC), Modified Polyethylene Terephthalate Glycol (PETG), Cellulose Acetate Butyrate (CAB); Semi-Crystalline Commodity Plastics that include Polyethylene (PE), High Density Polyethylene (HDPE), Low Density Polyethylene (LDPE or LLDPE), Polypropylene (PP), Polymethylpentene (PMP); Amorphous Engineering Thermoplastics that include Polycarbonate (PC), Polyphenylene Oxide (PPO), Modified Polyphenylene Oxide (Mod PPO), Polyphenylene Ether (PPE), Modified Polyphenylene Ether (Mod PPE), Thermoplastic Polyurethane (TPU); Semi-Crystalline Engineering Thermoplastics that include Polyamide (PA or Nylon), Polyoxymethylene (POM or Acetal), Polyethylene Terephthalate (PET, Thermoplastic Polyester), Polybutylene Terephthalate (PBT, Thermoplastic Polyester), Ultra High Molecular Weight Polyethylene (UHMW-PE); High Performance Thermoplastics that include Polyimide (PI, Imidized Plastic), Polyamide Imide (PAI, Imidized Plastic), Polybenzimidazole (PBI, Imidized Plastic); Amorphous High Performance Thermoplastics that include Polysulfone (PSU), Polyetherimide (PEI), Polyether Sulfone (PES), Polyaryl Sulfone (PAS); Semi-Crystalline High Performance Thermoplastics that include Polyphenylene Sulfide (PPS), Polyetheretherketone (PEEK); and Semi-Crystalline High Performance Thermoplastics, Fluoropolymers that include Fluorinated Ethylene Propylene (FEP), Ethylene Chlorotrifluroethylene (ECTFE), Ethylene, Ethylene Tetrafluoroethylene (ETFE), Polychlortrifluoroethylene (PCTFE), Polytetrafluoroethylene (PTFE), expanded Polytetrafluoroethylene (ePTFE), Polyvinylidene Fluoride (PVDF), Perfluoroalkoxy (PFA). Other commonly known medical grade materials include elastomeric organosilicon polymers, polyether block amide or thermoplastic copolyether (PEBAX) and metals such as stainless steel and nickel/titanium alloys. The above materials are intended for illustrative purposes only, and not as a limitation on the scope of the present disclosure. Suitable polymeric materials available for use are vast and too numerous to be listed herein and are known to those of ordinary skill in the art.

Medical devices can include, for example, stents, grafts, stent-grafts, filters, valves, occluders, markers, mapping devices, therapeutic agent delivery devices, prostheses, pumps, membranes, patches, meshes, bandages, and other endoluminal and implantable devices are frequently used to treat the anatomy (such as, for example, the vasculature) of patients. Such medical devices can be secured to the anatomy by one or more anchors. In some instances, the anchors are used to hold tissue to tissue as in the case of vascular dissection. In some cases, the anchors can be used to hold medical device to medical device as in the case of modular, multiple component stent-grafts. In some configurations, the anchor(s) can be separate from the medical device. In other configurations, the anchor(s) can be incorporated into and/or integral with the medical device

Anchors in accordance with the present disclosure provide a number of benefits over the prior art. For example, the tips of the anchors that engage tissue in a patient can be positioned in the tissue such that the depth of penetration of the tips is relatively easily controlled. Further, the tips can be everted in a direction and/or position that encourages tissue growth around the tips, subsequently reducing the danger of unintended damage to surrounding tissue caused by the anchor.

In this regard, a delivery system in accordance with the present disclosure can be used to deliver one or more anchors to the anatomy of a patient, wherein the anchor(s) can be incorporated into and/or integral with the medical device, or separate from the medical device, for example a simultaneously, sequentially, or previously implanted medical device.

Further, one or more anchors can used to provide treatment to the anatomy of a patient without an accompanying medical device. For example, one or more anchors can be used to close a wound or otherwise engage tissue to provide a therapeutic or beneficial effect. In other embodiments, one or more anchors can be used to provide a dock for later-deployed medical devices.

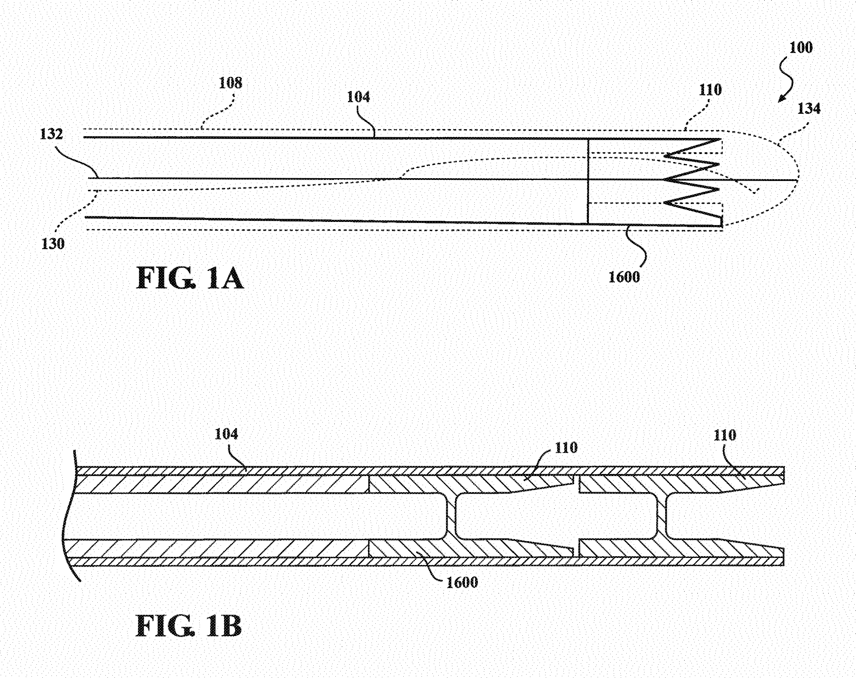

With reference now to Figure 1A, a delivery system 100 in accordance with the present disclosure is illustrated. Delivery system 100 comprises an elongate element 104 capable of delivering at least one anchor 110 to the anatomy of a patient. Delivery systems in accordance with the present disclosure can be configured to deliver at least one anchor 110, and/or both a medical device and at least one anchor 110 in conjunction. For example, as illustrated in Figure 1B, multiple anchors 110 can be consecutively deployed.

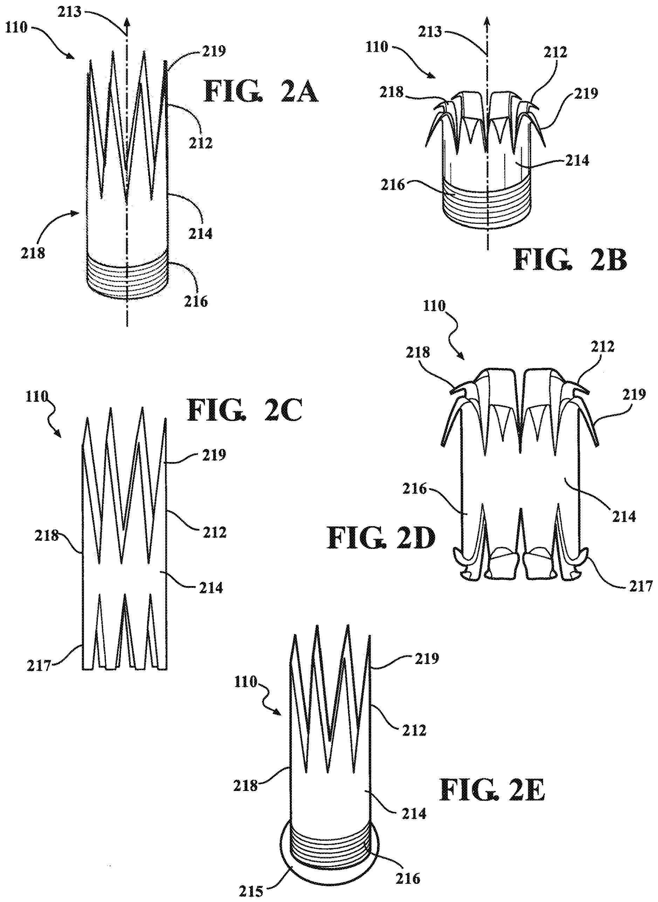

With reference to Figures 2A and 2B, an anchor 110 in accordance with the present disclosure is illustrated. Figure 2A illustrates anchor 110 prior to deployment and Figure 2B illustrates anchor 110 after deployment. Anchor 110 can vary in length, thickness, and diameter. In various embodiments, multiple anchors 110 are utilized to secure a medical device. Anchors 110 can have different length, thickness, and diameter from each other.

Anchor 110 can comprise a base portion 214. Base portion 214 can comprise, for example, a generally tubular shape with a central axis 213. Base portion 214 can further comprise an engagement portion 216. Engagement portion 216 may be configured to temporarily engage base portion 214 to a delivery system, such as a catheter and/or elongate element. Engagement portion 216 can comprise, for example, a threaded portion located on an outer diameter and/or an inner diameter of base portion 214. For example, engagement portion 216 can comprise of a number of threads along the outer diameter of base portion 214 configured to secure base portion 214 to a complementarily-threaded section on the inner surface of a tube and/or other member. In other configurations, engagement portion 216 comprises a number of threads along the inner diameter of base portion 214, such that base portion 214 engages with a complimentarily threaded section on the outer surface of an elongate element, tube, and/or other member, such as elongate element 104.

Engagement portion 216 can comprise a magnetic portion. For example, engagement portion 216 can comprise a magnetic material coupled to base portion 214. In such configurations, magnetic engagement portion 216 can temporarily couple base portion 214 to a corresponding magnetic portion of a delivery system. Once anchor 110 has been adequately deployed, magnetic engagement portion 216 can disengage from the delivery system. Although described as a threaded portion and a magnetic portion, any engagement portion that can temporarily engage base portion 214 and/or anchor 110 to a delivery system for deployment with the body of a patient is in accordance with the present disclosure.

Base portion 214 can comprise, wholly or in part, for example, a metal or metal alloy with shape-memory properties, such as Nitinol. In other embodiments, base portion 214 comprises a polymeric material capable of bending and/or everting to a predetermined shape or configuration upon deployment. In various embodiments, base portion 214 can comprise a shape that is laser cut from a tube, similar to, for example, a stent. Any material, including various metals and polymers, that is biocompatible and capable of anchoring a medical device to tissue is within the scope of the present disclosure.

In various embodiments, base portion 214 can be compressed or collapsed for delivery into the body of a patient. For example, base portion 214 can be compressed to fit inside a delivery catheter. In such configurations, when deployed, base portion 214 can be expanded, for example, by self-expansion or balloon-assisted expansion, to a larger diameter than the compressed diameter.

Anchor 110 can further comprise a plurality of anchor arms 212. In various embodiments, anchor arms 212 are positioned circumferentially on base portion 214. Anchor arms 212 of anchor 110 can comprise a shaft portion 218. Prior to deployment of anchor 110, shaft portion 218 is substantially parallel to central axis 213 of base portion 214.

In various embodiments, shaft portion 218 can be slit or lanced, such that shaft portion 218 can bend and/or evert during deployment of anchor 110. In such configurations, shaft portion 218 can evert during deployment such that anchor arms 212 bend up to, for example, 180 degrees, 360 degrees, or more from their original orientation. Such eversion can resemble a mushroom-style deformation, as each anchor arm 212 is bent to a similar degree and shape. For example, shaft portion 218 can evert such that each anchor arm 212 is substantially parallel to central axis 213.

Shaft portion 218 can comprise, for example, a material that allows shaft portion 218 to evert and/or bend in a predetermined manner. In various embodiments, shaft portion 218 comprises a metal or metal alloy with shape-memory properties, such as Nitinol. In other embodiments, shaft portion 218 comprises a polymeric material, such as a shape-memory polymer taught in U.S. Patent 7,498,385 to Swetlin et al, which is capable of bending and/or everting to a predetermined shape or configuration upon deployment. Typically, all components of anchor 110, including base portion 214, anchor arms 212, and shaft portion 218 are comprised of the same material. Any material, including various metals and polymers, that is biocompatible and capable of anchoring a medical device to tissue is within the scope of the present disclosure. Materials that can be appropriate include, but are not limited to full hard 316 stainless steel or L605 and Eligiloy and other materials which have self-deploying characteristics.

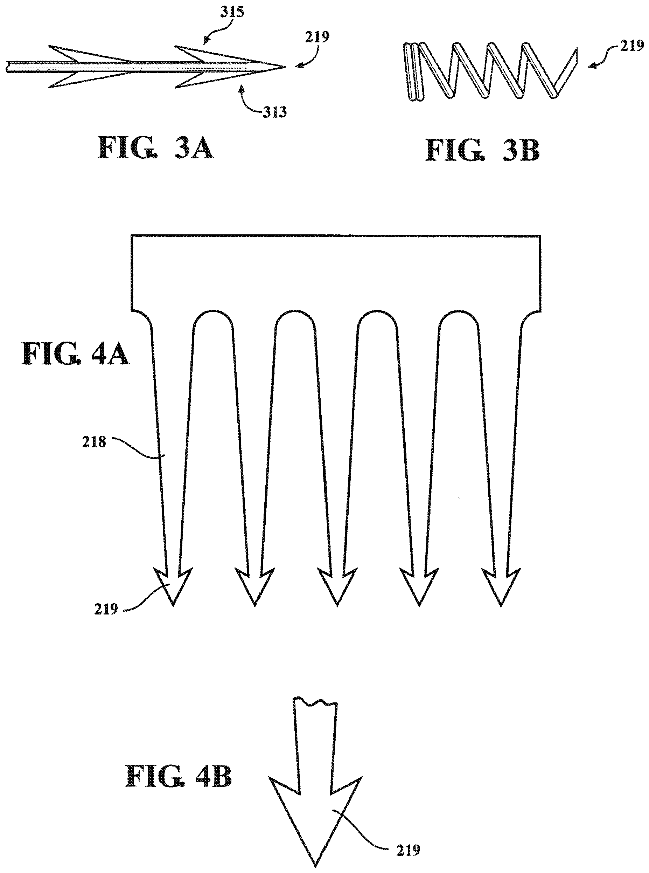

Anchor arms 212 can further comprise a tissue-penetrating point 219. In various embodiments, tissue-penetrating point 219 is located at the end of shaft portion 218. Tissue-penetrating point 219 can comprise a shape capable of penetrating tissue and securing anchor 110 to the anatomy of the patient. As illustrated in Figure 2A, tissue-penetrating point 219 can be, for example, substantially arrow-shaped. With reference to Figures 4A and 4B, multiple shaft portions 218, each equipped with a substantially arrow-shaped tissue-penetrating point 219 are illustrated.

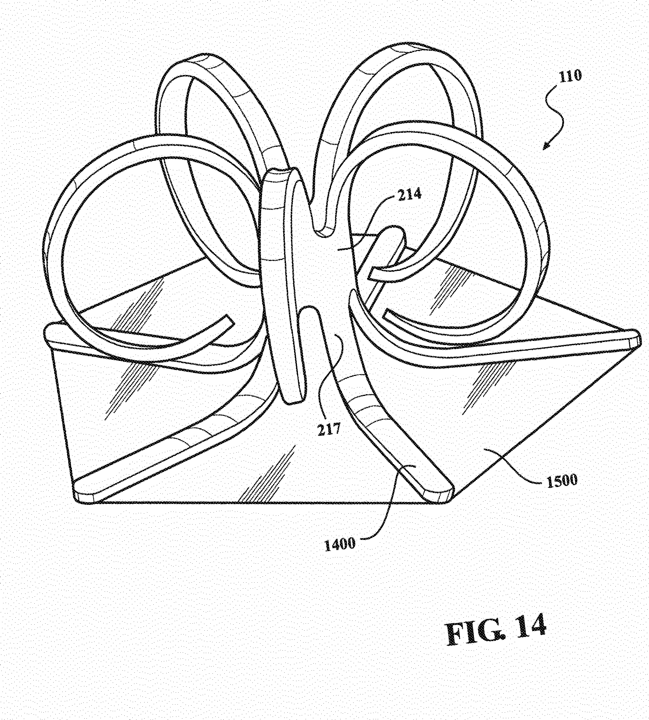

With reference to Figures 2C and 2D, an anchor 110 in accordance with the present disclosure can further comprise at least one flange element 217. In such configurations, flange elements 217 can evert after anchor arms 212 have everted, such that a medical device and/or the tissue engaged by anchor arms 212, shaft portion 218, and tissue-penetrating point 219 is sandwiched between anchor arms 212 and at least one flange element 217. In these embodiments, flange elements 217 can maintain proximal positioning between a medical device and the tissue engaged by anchor 110. In various embodiments, including the embodiment of Figure 14, the flange element 217 has one or more flange element arms 1400 having a portion of the one or more flange element arms substantially everting to a position approximately 90 degrees from the central axis of the base portion 214 of the anchor 110. The flange element 217 in some embodiments is atraumatic. As used herein, "atraumatic" refers to the flange element 217 and the flange element arms 1400 are designed to minimize or avoid penetration of tissue or a medical device or to avoid causing damage to tissue.

In various embodiments, and with reference to Figure 3A, tissue-penetrating point 219 can comprise a barb 313 with one or more fins 315. With reference to Figure 3B, tissue-penetrating point 219 can comprise a corkscrew-like configuration. Although described in a number of specific configurations, such as, for example, including arrow-shaped, barbed, and corkscrew-like, any configuration of tissue-penetrating point 219 that suitably engages with and secures a medical device to tissue is within the scope of the present disclosure. In various embodiments, the anchors 110 or one or more portions of the anchors such as the base portion 214, engagement portion 216, one or more anchor arms 212, one or more shaft portions 218, one or more tissue-penetrating points 219, one or more flange elements 217, and or one or more barbs 313 or fins 315 or combinations of any of the foregoing can be partially, substantially or wholly covered in a porous or fibrous biomaterial. The porous or fibrous biomaterial assists with the integration of the anchor 110 with the surrounding tissue. Biomaterials provide an open structure on the surface of the anchor 110 that is sufficiently large for cells to readily penetrate and promote ingrowth of both collagenous and vascular tissues for example. Porous structures for implantable devices sufficiently large to allow ingrowth and attachment of tissue can be achieved through a variety of means. Various technologies are able to deliver tailored open-celled structures with various pore sizes to fit the particular cell ingrowth applications. Such materials include fluorinated polymers and copolymers; expanded fluorinated polymers and copolymers; and woven, nonwoven, extruded or the like fibers including PGA:TMC copolymer (polyglycolic acid:trimethylenecarbonate copolymer) fibers. Any combination of these porous or fibrous biomaterials can be utilized in various embodiments. In various embodiments, expanded polytetrafluoroethylene (ePTFE) is used as a covering on one or more portions of the anchors 110. Various means for attaching the biomaterials can be utilized such as attaching with fluorinated ethylene propylene (FEP). Figure 14 illustrates a perspective view of an embodiment according to the present invention of an anchor 110 comprising a flange element 217 with flange element arms 1400 that are indexed to minimize interaction between the anchor arms 212 and the flange element arms 1400. Figure 14 also illustrates an embodiment with a fluorinated polymer, specifically an expanded polytetrafluoroethylene (ePTFE), biomaterial attached to the flange element as a cap 1500. The cap promotes tissue ingrowth and can be used to encase the flange element 217 of the anchor 110, thereby rendering it atraumatic.

In some embodiments, the anchor 110 or portions thereof can be covered with one or more bioactive agents to initiate a bio-response. Examples of such bioactive agents include antimicrobials, PGA:TMC , and anticoagulants such as Heparin. It should be noted that combinations of such bioactive agents can be applied even within the same anchor 110. For instance, in the case of an anchor 110 used to tack a vascular graft to the aortic wall, the anchor arms 212 can be coated with a substance which is known to generate a tissue healing response, while the flange elements 217 (and cap 1500) can be treated with Heparin, to mitigate clotting in the blood stream.

In various embodiments, the anchor 110 or any combination of portions thereof can be surface treated, for example sand blasted, coated by spraying or dipping for example to coat with a bioactive agent, and covered with a porous or fibrous biomaterial for example to initiate various desired bio-responses such as tissue ingrowth or anticoagulant responses,

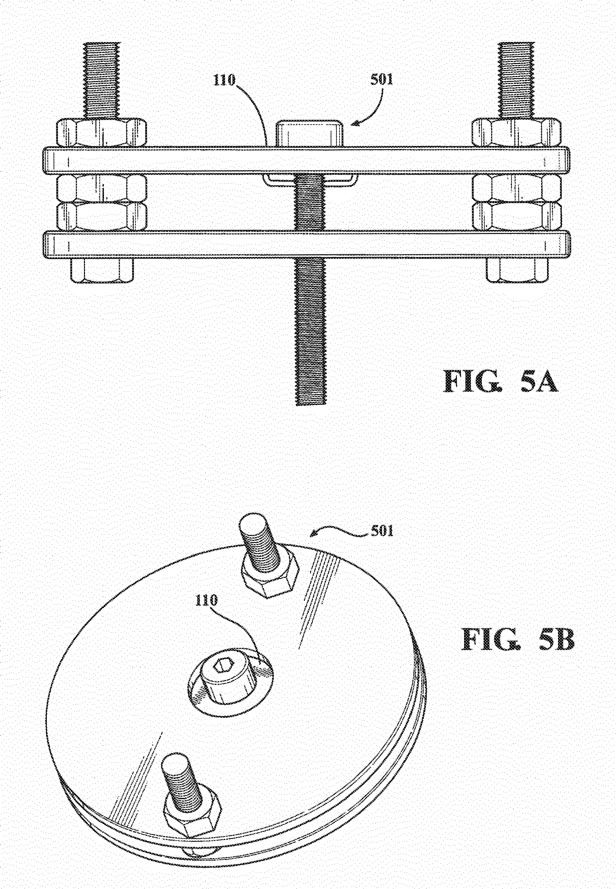

With reference to Figures 5A and 5B, anchors 110 can be formed by placing a laser-cut tube comprising, for example, Nitinol, in a form configured to conform the tube into the desired deployment configuration of anchor 110. For example, a tube is placed into form 501. Form 501 everts anchor arms 212 of the tube, creating the deployed configuration of anchor 110. In various embodiments, form 501 and anchor 110 are placed in an oven to heat treat and set the deployed configuration of anchor 110.

In embodiments in which anchor 110 is produced by heat treating, operating conditions of the heat treating process can be varied to produce different characteristics in anchor 110. For example, heat treating Nitinol at a relatively low temperature can produce a softer material, which may produce an anchor 110 that is easier to remove from tissue in the body of a patient. Treating Nitinol at a relatively higher temperature can produce a harder material, which can produce an anchor 110 that has improved grip and engagement with tissue of the patient

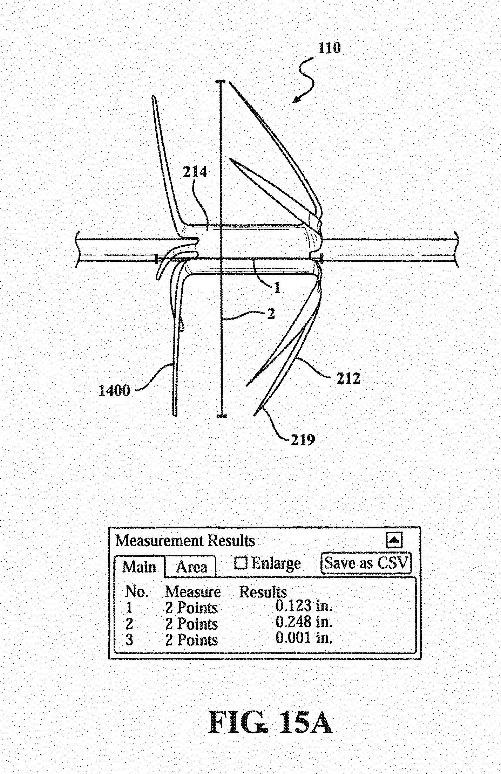

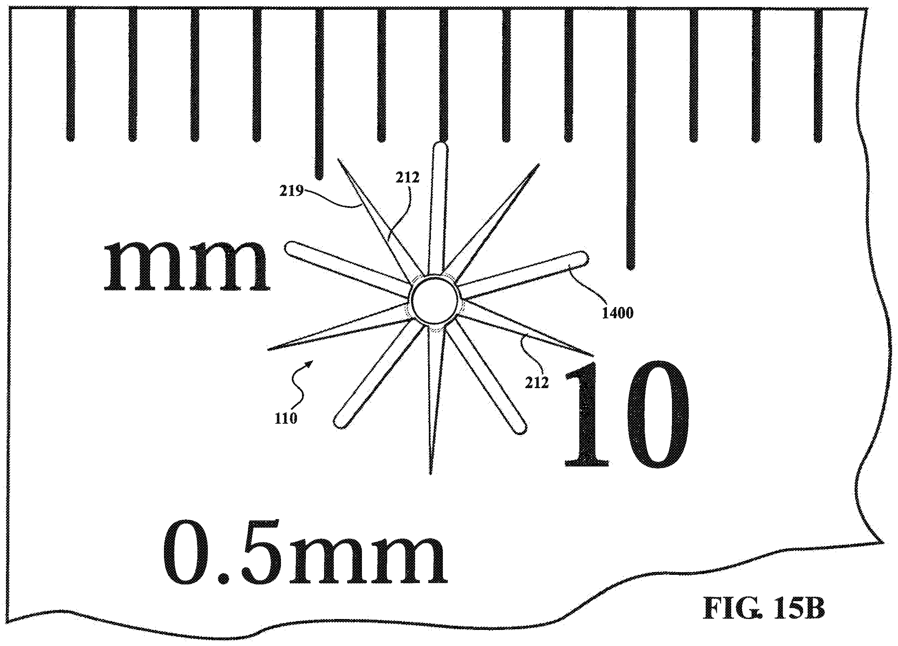

In an embodiment, with reference to Figures 15A and 15B, a laser-cut tube of Nitinol having an outer diameter of 0.076 cm (.030") was heat treated to result in an anchor 110 that can be delivered via lumen of a 1.3 mm (4 fr) catheter, elongate element. The anchor of Figures 15A and 15B has anchor arms 212 with tissue penetrating points 219 and atraumatic flange element arms 1400 with blunt ends as shown in Figure 15B. The anchor of Figures 15A and 15B when deployed has a length (shown as 1) of 0.30 cm (0.12 in.) and a width (shown as 2) of 0.64 cm (0.25 inches). With reference to Figure 15B, the anchor arms 212 and the flange element arms 1400 are indexed. Indexing avoids interference between the deploying anchor arms 212 and the flange element arms 1400. Both deploy away from the central axis of the base portion 214.

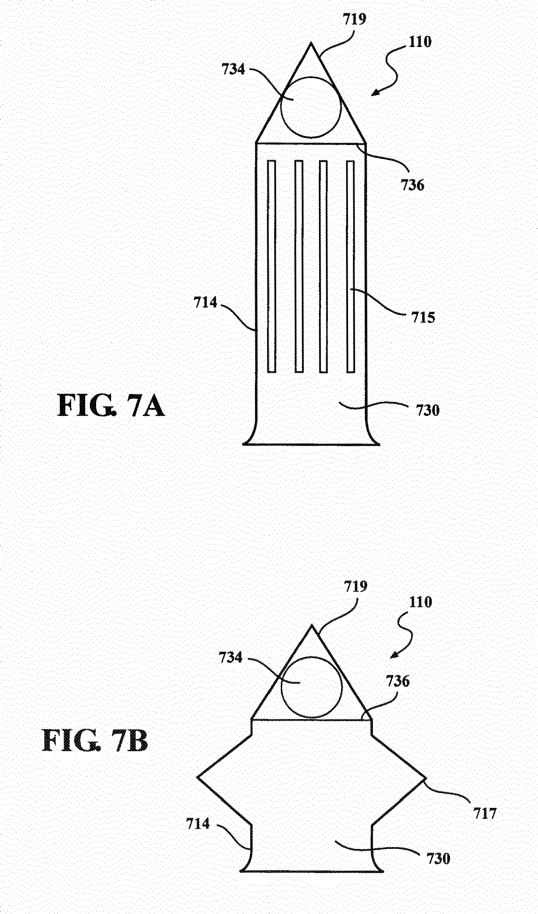

In various embodiments, with reference to Figure 7A, anchor 110 comprises a substantially cylindrical body 714 and a tissue-penetrating point 719. Cylindrical body 714 can further comprise a plurality of slots 715.

Anchor 110 can further comprise a tip 734, activation wire 730, and retention element 736. In such configurations, tip 734 can comprise a ball affixed (by, for example, welding) to activation wire 730. When tension is applied to tip 734 by activation wire 730, tip 734 is drawn towards the base of anchor 110. Because tip 734 is held in place inside of anchor 110 by retention element 736, the force applied to tip 734 causes cylindrical body 714 to partially collapse.

As illustrated in Figure 7B, partial collapse of cylindrical body 714 can cause the material between slots 715 to expand radially, creating a number of tissue-engaging points 717. Once sufficient engagement between tissue and anchor 110 is achieved, anchor 110 can be disengaged from elongate element 104. In various embodiments, retention element 736 can comprise, for example, an element that disengages when sufficient force is applied. In such configurations, sufficient force to disengage retention element 736 is higher than the force required to partially collapse cylindrical body 714. In other embodiments, retention element 736 comprises member having a hole, such that as cylindrical body 714 partially collapses, the hole of retention element 736 increases in diameter. After sufficient collapsing of cylindrical body 714, tip 734 can pass through retention element 738 and be removed from the patient.

As discussed previously, anchors 110 can be utilized and/or deployed independently from medical devices to secure medical devices to the anatomy of a patient, for example a simultaneously, sequentially or previously implanted medical devices. For example, with reference to Figure 2E, one or more anchors 110 can be utilized to secure a properly positioned graft member to the wall of a vessel, such as the aorta. In such configurations, the graft member can be positioned within the vessel, and anchors 110 can be deployed such that the end of the anchor passes through the graft member and engages the vessel. Flared segment 215, which can comprise a portion having a larger diameter than the diameter of base portion 214, such that flared segment 215 can assist in maintaining proper positioning of the graft member relative to the vessel.

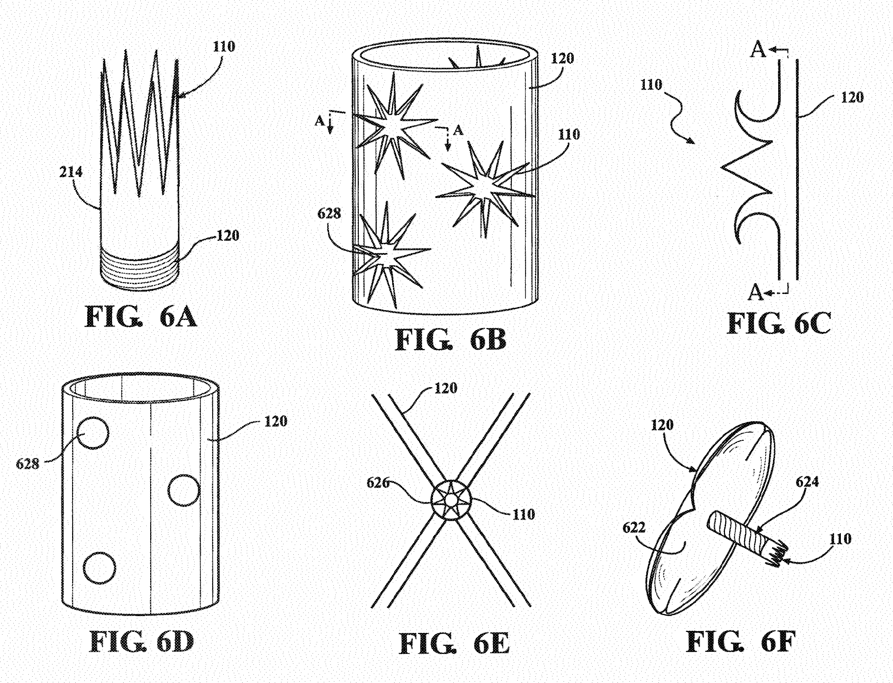

Figure 6D illustrates another embodiment in which an anchor or multiple anchors 110 can be used to secure medical device 120, such as a stent or stent graft, to the anatomy of a patient. In such embodiments, medical device 120 is a stent or stent graft with one or more holes 628. Holes 628 can be configured, for example, to allow an anchor 110, as illustrated in Figure 2E, to pass through the stent or stent graft and engage the anatomy of the patient. In such configurations, flared segment 215 can comprise a larger diameter or cross sectional profile than holes 628, such that anchor 110 is capable of securing medical device 120 through holes 628. Such engagement can retain the proper positioning of medical device 120 within the anatomy.

In other embodiments, anchor 110 can be incorporated into and/or integral with medical device 120. Figures 6A, 6B, 6C, 6E, and 6F illustrate anchors 110 that are incorporated into and/or integral with medical devices 120. For example, as illustrated in Figure 6A, a medical device 120 can comprise a drug-eluting button that can be incorporated into base portion 214 of anchor 110. In other embodiments, medical device 120 can comprise an intercardiac device, such as a device designed to transmit electrical energy to tissue of the heart.

In various embodiments, for example as illustrated in Figures 6B and 6C, one or more anchors 110 can be incorporated into medical device 120. For example, medical device 120 can comprise a metal tube with one or more holes 628. The shape of holes 628 can comprise one or more points 629. In such configurations, points 629 comprise anchors 110. Medical device 120 can be deployed into a vessel, and once properly positioned, anchors 110 can be deployed such that points 629 evert, as illustrated in 6C, and engage with the vessel wall.

In various embodiments, for example as illustrated in Figures 6E, anchors 110 can connect directly to and deploy concurrently with medical device 120. For example, medical device 120 can comprise a stent that is constructed from metal rings joined together at apices. In such embodiments, one or more anchors 110 are positioned at one or more of the apices 626 of the stent of medical device 120. When deployed, anchors 110 secure the stent of medical device 120 to the anatomy of the patient. In such embodiments, anchors 110 can remain connected to medical device 120 after deployment, maintaining engagement between medical device 120 and the anatomy of the patient. Although anchors 110 are described as located at apices 626, anchors can be located anywhere along the stent of medical device 120.

With reference to Figure 6F, medical device 120 can comprise a modified occluding device, such as a single-disk occluding device for use in reducing the volume of a left atrial appendage of the heart. In such embodiments, one of the two disks can be removed and replaced with an anchor 110. A connecting element 624 can connect an occluding disk 622 to anchor 110. Anchor 110 can then properly position the remaining occluding disk 622 relative to the left atrial appendage, obviating the need for the second disk. Although described in connection with a single-disk occluding device, medical device 120 can be any device that can combine with or incorporate into and/or be formed integral with anchor 110 and deployed to the anatomy of a patient.

With reference back to Figure 1A, delivery system 100 can further comprise a sheath 108, such as, for example, a delivery sheath. Sheath 108 can be configured to surround anchor or anchors 110, a medical device, or both anchors 110 and a medical device. Sheath 108 can assist in the deployment of anchors 110 and/or a medical device. The portion 1600 of the elongate element 104, catheter or sheath, as shown in Figures 1A and 1B, that houses the anchor or anchors when loaded and during unloading can be comprised of various materials or composites to minimize interaction between the anchors and the inner diameter of the elongate element. These materials include, but are not limited to, metals or alloys such as Nitinol and stainless steel, high durometer plastics/polymers, radiopaque cuffs/bands, or any similar biocompatible material. Alternatively, the inner diameter of that portion of the elongate element can be coated or have an insert of such materials to minimize the interaction of the inner diameter of that portion with the anchors. Use of these materials in the loading and delivery portion of the elongate element or catheter can minimize particulation and improve delivery of the anchors.

Delivery system 100 may further comprise an activation wire 132. Optionally, delivery system can further comprise a tip 134. As will be discussed later in greater detail, activation wire 132 and/or tip 134 can assist in the deployment of anchors 110 and/or medical device 120.

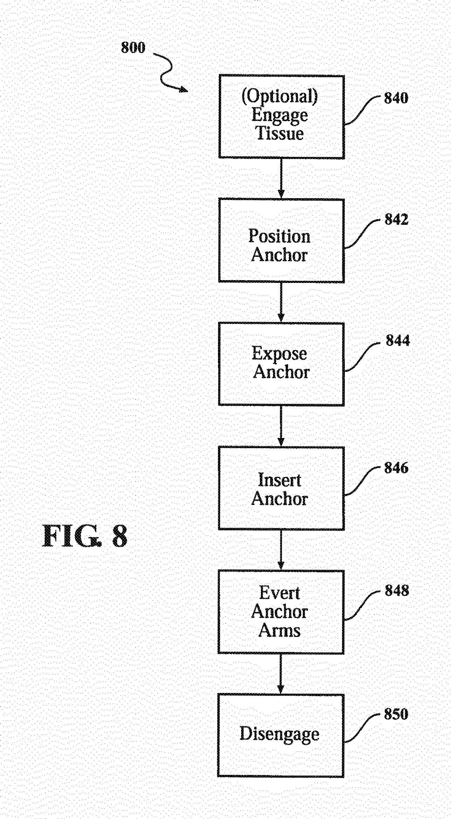

With reference to Figure 8, an anchor deployment method 800 in accordance with the present disclosure is illustrated. Delivery system 100 can be used to deliver and deploy one or more anchors 110 according to anchor deployment method 800.

Anchor deployment method 800 comprises an optional engage tissue step 840. For example, engage tissue step 840 can comprise using a fixation wire 130 to temporarily engage the tissue of the anatomy by entering the tissue. Fixation wire 130 can comprise a point that is capable of piercing and embedding in tissue. Once embedded, the point of fixation wire 130 can allow the operator to position elongate element 104, sheath 108, and anchor 110 for deployment. Although described in connection with fixation wire 130, any device or tool that allows for temporary engagement of tissue and positioning of sheath 108, and/or anchor 110 is within the scope of the present disclosure.

Anchor deployment method 800 may further comprise a position anchor step 842. Position anchor step 842 can comprise elongate element 104 and sheath 108 such that anchor 110 is located in proximity to the desired deployment location in the anatomy of the patient. For example, position anchor step 842 can comprise using fixation wire 130 to assist in directing elongate element 104, sheath 108, and anchor 110 to the proper position for deployment of anchor 110.

Anchor deployment method 800 further comprises an expose anchor step 844. Expose anchor step 844 can comprise preparing anchor 110 for insertion into the tissue of the anatomy. For example, expose anchor step 844 can comprise withdrawing sheath 108 from anchor 110 and exposing at least a portion of anchor 110.

Anchor deployment method 800 may further comprise an insert anchor step 846. Insert anchor step 846 can comprise, for example, using delivery system 100 to insert anchor arms 212 of anchor 110 into the tissue of the anatomy. In such configurations, tissue-penetrating point 219 of anchor arm 212 can allow anchor arms 212 to effectively penetrate and engage the tissue at the desired location within the anatomy.

Anchor deployment method 800 further comprises an evert anchor arms step 848. Evert anchor arms step 848 can comprise bending and/or everting shaft portion 218 of anchor arms 212. For example, shaft portions 218 of anchor arms 212 can be bent such that anchor arms 212 are in a mushroom-shaped configuration, and at least a segment of shaft portion 218 is substantially parallel to central axis 213. Anchor arms 212 comprise a shape memory metal alloy, such as Nitinol, which has been pre-set to the desired everted configuration. As anchor 110 is inserted into the tissue of the patient, anchor arms 212 return to the everted pre-set configuration.

Evert anchor arms step 848 comprises bending and/or everting anchor arms 212 using tip 134 of delivery system 100. For example, tip 134 can be located concentrically to and extend past tissue-penetrating points 219 of anchor arms 212. Tip 134 can comprise a portion that is larger than the diameter of anchor 110. When anchor 110 is engaged in the tissue at the desired location in the anatomy, tip 134 can be retracted, exerting pressure on and causing plastic deformation of anchor arms 212, thereby bending and/or everting shaft portions 218. However, any manner of bending and/or everting anchor arms 212 to cause a sufficiently strong and secure engagement between anchor 110 and the desired tissue is within the scope of the present disclosure.

Anchor deployment method 800 further comprises a disengage step 850. Disengage step 850 can comprise, for example, separating anchor 110 from delivery system 100. Anchor 110 may be uncoupled from elongate element 104 by rotating elongate element 104 until the threads of anchor 110 and elongate element 104 disengage from each other. Anchor 110 can be coupled to elongate element 104 by other methods, such as, for example, a tension fit. In such configurations, disengage step 850 comprises uncoupling anchor 110 and elongate element 104 by the appropriate method.

Disengage step 850 can further comprise removing any temporary engagement device or tool from the tissue of the anatomy. For example, when fixation wire 130 is used in engage tissue step 840, disengage step 850 can comprise removing fixation wire 130 from the tissue of the anatomy.

Anchor deployment method 800 can be used in conjunction with the delivery of a variety of different medical devices 120. For example, as described in relation to anchor 110 of Figure 2A, anchor deployment method 800 can be used to deliver an integrated anchor 110 and medical device 120. Multiple integrated anchors 110 and medical devices 120 can be delivered to different locations within the anatomy using the same delivery system 100.

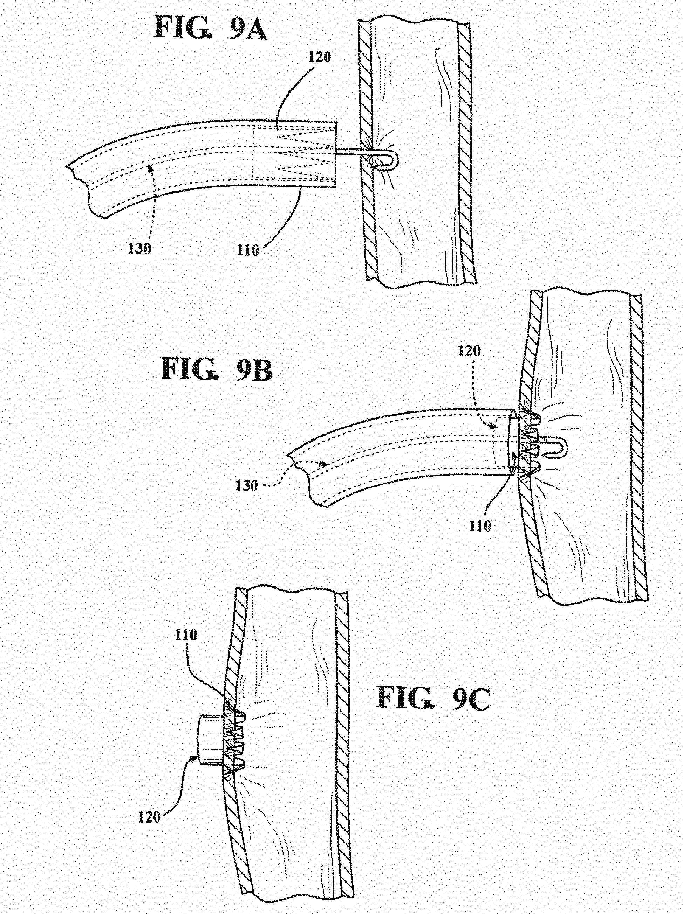

For example, with reference to Figures 9A-9C, the deployment of a medical device 120 integrated with anchor 110 is illustrated. For example, Figure 9A illustrates anchor 110, and fixation wire 130 during engage tissue step 840 of anchor deployment method 800. Figure 9B illustrates such a system during an evert anchor arms step 848 of anchor deployment method 800. Figure 9C illustrates anchor 110, and fixation wire 130 after the completion of disengage step 850 of anchor deployment method 800.

A plurality of anchors 110 can be deployed to secure medical device 120 to the anatomy of the patient. For example, as described in relation to Figure 6B, medical device 120 can comprise holes 628 configured to allow anchors 110 to pass through from the inside of medical device 120 and contact the tissue of the anatomy. In such configurations, anchors 110 can be deployed individually, either in sequence or simultaneously.

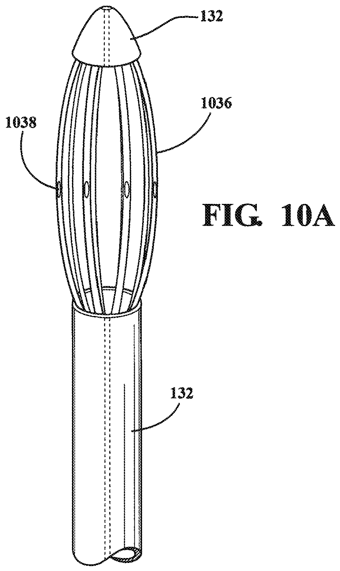

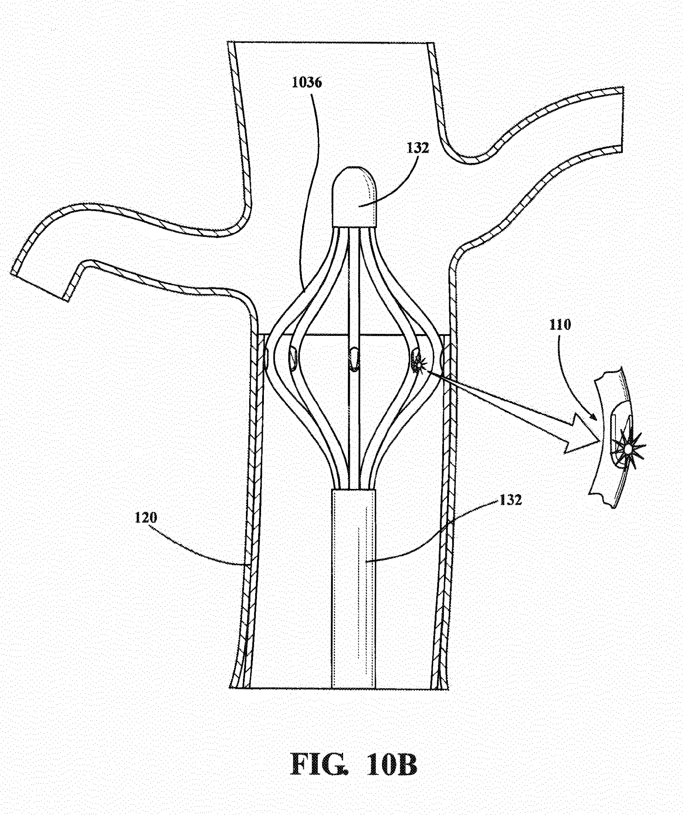

For example, as illustrated in Figures 10A and 10B, a number of anchors 110 can be deployed simultaneously to secure medical device 120 to the tissue of the anatomy. Figure 10A illustrates a delivery system comprising a series of hypotubes 1036, each comprising a trap door 1038. Each of a plurality of anchors 110 is stored in hypotubes 1036. Each trap door 1038 may correspond to a position along medical device 120 to be secured to the anatomy of the patient. For example, the positions of trap doors 1038 can correspond with locations of holes 628 of medical device 120 illustrated in Figure 6B.

As illustrated in Figure 10B, activation wire 132 can be configured such that when tension is applied to it, hypotubes 1036 compress longitudinally and expand perpendicularly. Further, tension applied to activation wire 132 exposes anchors 110 to the anatomy of the patient. Applying sufficient tension to activation wire 132 may causes anchors 110 to engage with the tissue of the anatomy simultaneously. After anchors 110 have sufficiently engaged the anatomy, tension can be released from activation wire 132, and anchors 110 thereby disengaged.

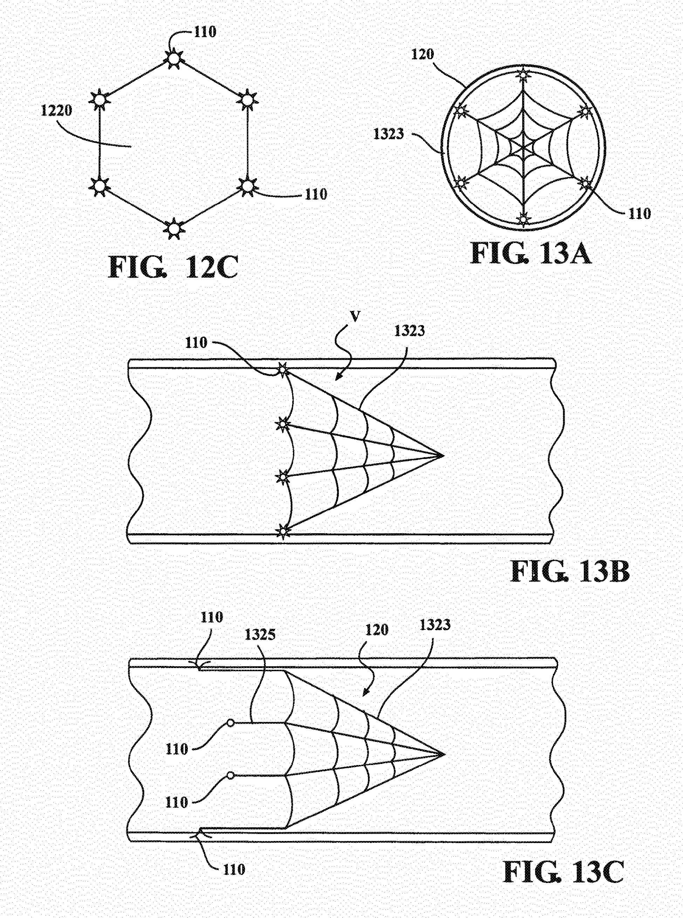

With reference to Figures 13A-13C, medical device 120 can comprise a net 1323 and one or more anchors 110. As illustrated in Figure 13A, net 1323 can comprise a substantially round cross section having a plurality of anchors 110 disposed around the perimeter of net 1323. When deployed in the vasculature of a patient, as illustrated in Figures 13B and 13C, net 1323 can have a substantially cone-shaped profile that allows it to trap and retain debris inside of a vessel. Such a configuration allows for the temporary, semi-permanent, or permanent installation of net 1323 in a particular vessel.

Net 1323 may comprise a plurality of biocompatible, polymeric threads. For example, net 1323 can comprise a plurality of ePTFE threads joined to form a substantially round cross section and a substantially cone-shaped profile. However, net 1323 can comprise any material that can trap and retain debris in the vasculature of a patient.

Net 1323 and anchors 110 can be deployed as illustrated in Figures 10A and 10B. With momentary reference to these figures, anchors 110 are simultaneously deployed through hypotubes 1038, engaging in the vasculature of the patient and providing anchoring for net 1323. Benefits of such deployment of net 1323 include allowing for bi-directional deployment and retrievability, reduced vessel wall penetration due to reduced outward radial force against the vessel during deployment, low or no tilting of net 1323 relative to the vessel wall, and limited or no fracture of net 1323. While the illustrated deployment method provides a number of benefits, any deployment method that successfully implants, temporarily, semi-permanently, or permanently, net 1323 and anchors 110 within the vasculature of a patient is within the scope of the present disclosure.

As illustrated in Figure 13C, medical device 120 may comprise net 1323, anchors 110, and a plurality of tethers 1325. In such configurations, one or more tethers 1325 extend from a location along the perimeter of net 1323. Each tether 1325 engages with an anchor 110. Tethers 1325 may be substantially the same length as one another. Tethers 1325 may comprise at least two different lengths, such that at least two tethers 1325 are not the same length as one another. Although described in connection with tethers, any manner of utilizing anchors 110 to affix net 1323 to the vasculature of a patient is within the scope of the present disclosure.

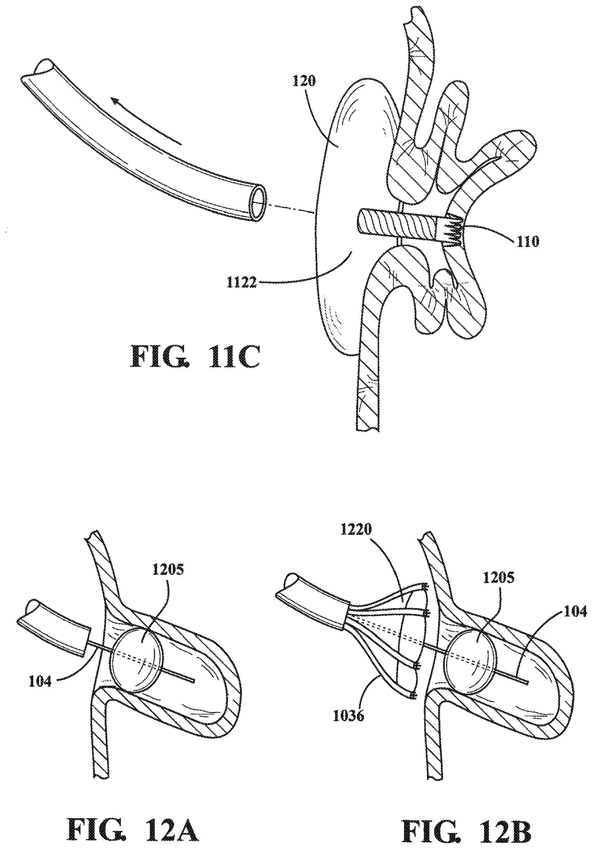

Multiple anchors 110 can be deployed in sequentially or simultaneously to secure a device, such as an occluder or a patch, to seal off, for example, a vessel, portion of a vessel, or left atrial appendage. For example, with reference to Figures 12A-12C, patch 1220 can be secured to the tissue of a patient by multiple anchors 110. As illustrated in Figure 12A, elongate element 104, and balloon 1205 can interface with a treatment area, such as, for example, a left atrial appendage. Balloon 1205 can assist in positioning elongate element 104 relative to the left atrial appendage. As illustrated in Figure 12B, a number of hypotubes 1036 can be used to deliver multiple anchors 110. Each hypotube 1036 can deliver one anchor 110 to the tissue of the treatment area.

Multiple anchors 110 can simultaneously engage with and secure patch 1220 to the tissue of the heart surrounding the left atrial appendage. As illustrated in Figure 12C, patch 1220 can be secured at numerous points along the body, including the perimeter, of patch 1220, thereby sealing off the left atrial appendage.

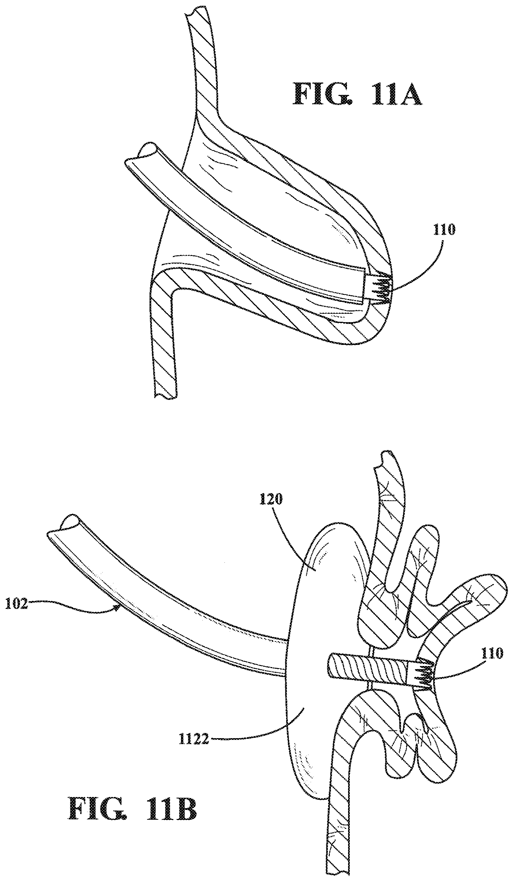

As described in relation to Figure 6C, one or more of a combination medical device 120 and anchor 110 can be deployed. For example, as illustrated in Figures 11A-11C, medical device 120 can comprise an occluding disk 1122 coupled to at least one anchor 110. In such configurations, one or more anchors 110 assist in positioning and engagement of medical device 120.

Figure 11A illustrates a delivery system used to position and deploy anchor to a desired treatment area, such as, for example, the left atrial appendage of a patient. Anchors 110 can be deployed such that the anchor engages a portion of the left atrial appendage. Once anchor 110 has engaged the left atrial appendage, the delivery system can be withdrawn, which reduces the volume of the left atrial appendage.

As illustrated in Figure 11B, medical device 120 can be deployed from the delivery system after the left atrial appendage has been properly engaged and the volume sufficiently reduced. Subsequently, as illustrated in Figure 11C, medical device 120 can be released from the delivery system, allowing occluding disk 1122 to engage with the left atrial wall to maintain the reduced volume of and prevent blood flow into the left atrial appendage.

In various embodiments, anchors 110 can be removed from the tissue of a patient. For example, elongate element 104 can be used to remove one or more anchors 110. In such configurations, elongate element 104 can be reengaged with anchor 110 by, for example, coupling the threaded portion of elongate element 104 with the complimentarily threaded portion of anchor 110. Elongate element 104 can then be retracted, causing anchor arms 212 to disengage with the tissue and allowing for the removal of anchor 110.

Although various particular embodiments are particularly described herein, any combination of anchor 110 and medical device 120 that provides a desired treatment to a patient is within the scope of the present disclosure. Further, any order of deployment that provides suitable positioning and engagement of medical device 120 with the anatomy of the patient is within the scope of the present disclosure. Specifically, one or more anchors 110 and one or more medical devices 120 can be deployed using a single or multiple delivery systems 100, in any order of deployment that achieves the desired result. For example, medical devices 120 can be deployed before, during, or after the deployment of one or more anchors 110, and vice versa.

It will be apparent to those skilled in the art that various modifications and variations can be made in the present disclosure. Thus, it is intended that the present disclosure cover the modifications and variations of this disclosure provided they come within the scope of the appended claims.

Likewise, numerous characteristics and advantages have been set forth in the preceding description, including various alternatives together with details of the structure and function of the devices and/or methods. The disclosure is intended as illustrative only and as such is not intended to be exhaustive. It will be evident to those skilled in the art that various modifications can be made, especially in matters of structure, materials, elements, components, shape, size and arrangement of parts including combinations within the principles of the disclosure, to the full extent indicated by the broad, general meaning of the terms in which the appended claims are expressed. To the extent that these various modifications do not depart from the scope of the appended claims, they are intended to be encompassed therein.

## Claims

1. An anchor (110) for tissue attachment or medical device to tissue attachment, comprising
a generally tubular base portion (214);
at least one anchor arm (212) integrally formed with the generally tubular base portion (214); and
a plurality of flange element arms (1400) integrally formed with the generally tubular base portion (214);
wherein the at least one anchor arm (212) comprises a tissue-penetrating point (219) and a shaft portion (218); and
wherein each of the at least one anchor arm (212) and the plurality of flange element arms are adapted to radially actuate upon deployment, and
**characterized in that** a cap (1500) of fluorinated polymer biomaterial providing an open structure promoting tissue ingrowth is attached to the flange element arms (1400).

2. The anchor (110) according to claim 1, wherein the at least one flange element arm is atraumatic.

3. The anchor (110) according to any one of claims 1 or 2, wherein each of the at least one flange element arm (1400) are adapted to substantially evert to 90 degrees from the central axis of the base portion upon deployment.

4. The anchor (110) according to any one of claims 1 to 3, wherein the anchor arms (212) and the flange element arms (1400) are indexed to minimize interaction between the anchor arms (212) and the flange element arms (1400).

5. The anchor (110) according to any one of claims 1 to 4, wherein tissue-penetrating point (219) comprises a barb (313) with one or more fins (315), a corkscrew-like configuration or is arrow-shaped.

6. The anchor (110) according to any one of claims 1 to 5, wherein the shaft portion (218) of the at least one anchor arm (212) is slit or lanced such that shaft portion (218) can bend and/or evert during deployment of anchor (110).

7. The anchor (110) according to claim 6, wherein the shaft portion (218) is adapted to evert during deployment such that the anchor arms (212) bend up to 180 degrees, 360 degrees, or more from their original orientation.

8. A medical device incorporating or being integral with the anchor (110) according to any one of claims 1 to 7.

## Patentansprüche

1. Anker (110) zur Gewebeanbringung oder medizinische Vorrichtung zur Gewebeanbringung, umfassend
einen im Allgemeinen rohrförmigen Basisabschnitt (214);
mindestens einen Ankerarm (212), der einstückig mit dem im Allgemeinen rohrförmigen Basisabschnitt (214) ausgebildet ist; und
eine Vielzahl von Flanschelementarmen (1400), die einstückig mit dem im Allgemeinen rohrförmigen Basisabschnitt (214) ausgebildet sind;
wobei der mindestens eine Ankerarm (212) eine Gewebedurchdringungsspitze (219) und einen Schaftabschnitt (218) umfasst; und
wobei jeder von dem mindestens einen Ankerarm (212) und den mehreren Flanschelementarmen dazu ausgelegt sind, sich beim Entfalten radial zu bewegen, und
**dadurch gekennzeichnet, dass** eine Kappe (1500) aus fluoriertem Polymer-Biomaterial, die eine offene, ein Einwachsen von Gewebe fördernde Struktur bereitstellt, an den Flanschelementarmen (1400) angebracht ist.

2. Anker (110) nach Anspruch 1, wobei der mindestens eine Flanschelementarm atraumatisch ist.

3. Anker (110) nach einem der Ansprüche 1 oder 2, wobei jeder des mindestens einen Flanschelementarms (1400) dazu ausgelegt ist, sich beim Entfalten im Wesentlichen bis 90 Grad von der Mittelachse des Basisabschnitts umzustülpen.

4. Anker (110) nach einem der Ansprüche 1 bis 3, wobei die Ankerarme (212) und die Flanschelementarme (1400) indexiert sind, um die Wechselwirkung zwischen den Ankerarmen (212) und den Flanschelementarmen (1400) zu minimieren.

5. Anker (110) nach einem der Ansprüche 1 bis 4, wobei die Gewebedurchdringungsspitze (219) einen Widerhaken (313) mit einer oder mehreren Rippen (315), eine korkenzieherartige Konfiguration umfasst oder pfeilförmig ist.

6. Anker (110) nach einem der Ansprüche 1 bis 5, wobei der Schaftabschnitt (218) des mindestens einen Ankerarms (212) derart geschlitzt oder eingeschnitten ist, dass sich der Schaftabschnitt (218) während des Entfaltens des Ankers (110) biegen und/oder umstülpen kann.

7. Anker (110) nach Anspruch 6, wobei der Schaftabschnitt (218) dazu ausgelegt ist, sich während des Entfaltens derart umzustülpen, dass sich die Ankerarme (212) von ihrer ursprünglichen Ausrichtung bis zu 180 Grad, 360 Grad oder mehr biegen.

8. Medizinische Vorrichtung, die den Anker (110) nach einem der Ansprüche 1 bis 7 enthält oder mit diesen einstückig ist.

## Revendications

1. Dispositif d'ancrage (110) pour fixation tissulaire ou dispositif médical relié à une fixation tissulaire, comprenant
une partie de base généralement tubulaire (214) ;
au moins un bras d'ancrage (212) faisant partie intégrante de la partie de base généralement tubulaire (214) ; et
une pluralité de bras d'élément bride (1400) faisant partie intégrante de la partie de base généralement tubulaire (214) ;
ledit au moins un bras d'ancrage (212) comprenant un point (219) pénétrant dans le tissu et une partie tige (218) ; et
chacun dudit au moins un bras d'ancrage (212) et de la pluralité de bras d'élément bride étant conçu pour s'actionner radialement lors du déploiement, et
**caractérisé en ce qu'**un capuchon (1500) de biomatériau polymère fluoré qui fournit une structure ouverte favorisant la croissance tissulaire est fixé aux bras d'élément bride (1400).

2. Dispositif d'ancrage (110) selon la revendication 1, ledit au moins un bras d'élément bride étant atraumatique.

3. Dispositif d'ancrage (110) selon l'une quelconque des revendications 1 ou 2, chacun dudit au moins un bras d'élément bride (1400) étant conçu pour sensiblement se retourner d'un angle de 90 degrés par rapport à l'axe central de la partie de base lors du déploiement.

4. Dispositif d'ancrage (110) selon l'une quelconque des revendications 1 à 3, lesdits bras d'ancrage (212) et lesdits bras d'élément bride (1400) étant indexés pour minimiser l'interaction entre les bras d'ancrage (212) et les bras d'élément bride (1400).

5. Dispositif d'ancrage (110) selon l'une quelconque des revendications 1 à 4, ledit point (219) pénétrant dans le tissu comprenant un ardillon (313) doté d'une ou plusieurs ailettes (315), une configuration de type tire-bouchon ou étant en forme de flèche.

6. Dispositif d'ancrage (110) selon l'une quelconque des revendications 1 à 5, ladite partie tige (218) dudit au moins un bras d'ancrage (212) étant fendue ou percée de sorte que la partie tige (218) puisse être cintrée et/ou retournée pendant le déploiement du dispositif d'ancrage (110).

7. Dispositif d'ancrage (110) selon la revendication 6, ladite partie tige (218) étant conçue pour se retourner pendant le déploiement de sorte que les bras d'ancrage (212) se cintrent jusqu'à 180 degrés, 360 degrés ou plus par rapport à leur orientation originale.

8. Dispositif médical incorporant ou faisant partie intégrante du dispositif d'ancrage (110) selon l'une quelconque des revendications 1 à 7.
